(19) Europäisches Patentamt
European Patent Office
Office européen des brevets

(11) EP 2 409 699 B1

(12) # EUROPEAN PATENT SPECIFICATION

(45) Date of publication and mention of the grant of the patent:
**30.04.2014 Bulletin 2014/18**

(21) Application number: **10382204.5**

(22) Date of filing: **23.07.2010**

(51) Int Cl.:
***A61K 31/506*** *(2006.01)*
***A61K 31/724*** *(2006.01)*
***A61P 31/10*** *(2006.01)*
***A61K 31/198*** *(2006.01)*
***A61K 31/4178*** *(2006.01)*

(54) **Stable compositions of voriconazole**

Stabile Voriconazole-enthaltende Zusammensetzungen

Compositions stables de voriconazole

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(43) Date of publication of application:
**25.01.2012 Bulletin 2012/04**

(73) Proprietor: **Combino Pharm, S.L.**
**08970 Sant Joan Despí, Barcelona (ES)**

(72) Inventors:
- **Puigvert Colomer, Marina**
  **E-08014 Barcelona (ES)**
- **Lloret Pérez, Sergio**
  **E-08030 Barcelona (ES)**

(74) Representative: **ABG Patentes, S.L.**
**Avenida de Burgos, 16D**
**Edificio Euromor**
**28036 Madrid (ES)**

(56) References cited:
**EP-A1- 0 440 372**
**WO-A1-98/58677**
**WO-A2-2006/065726**
**US-A1- 2002 177 611**

- **XIULAN HUANG ET AL: "Development and Validation of an Accurate LC Method for the Quantitative Determination of Voriconazole in a New Emulsion Formulation", CHROMATOGRAPHIA ; AN INTERNATIONAL JOURNAL FOR RAPID COMMUNICATION IN CHROMATOGRAPHY, ELECTROPHORESIS AND ASSOCIATED TECHNIQUES, VIEWEG VERLAG, WI, vol. 68, no. 7-8, 24 July 2008 (2008-07-24), pages 649-652, XP019637886, ISSN: 1612-1112, DOI: DOI:10.1365/S10337-008-0737-9**
- **SRINUBABU ET AL: "Development and validation of a HPLC method for the determination of voriconazole in pharmaceutical formulation using an experimental design", TALANTA, ELSEVIER, AMSTERDAM, NL, vol. 71, no. 3, 30 January 2007 (2007-01-30), pages 1424-1429, XP005864596, ISSN: 0039-9140, DOI: DOI: 10.1016/J.TALANTA.2006.04.042**
- **"Pharmaceutical formulation containing voriconazole", IP.COM JOURNAL, IP.COM INC., WEST HENRIETTA, NY, US, 16 July 2008 (2008-07-16), XP013125809, ISSN: 1533-0001**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 409 699 B1

**Description**

BACKGROUND OF THE INVENTION

The present invention is concerned with compositions comprising voriconazole, in particular aqueous solutions and lyophilized powders, and improved formulation processes therefor.

Voriconazole is an active pharmaceutical substance with an empirical formula of $C_{16}H_{14}F_3N_5O$ and a molecular weight of 349.31. Voriconazole is the international commonly accepted non-proprietary name for (2*R*,3*S*)-2-(2,4-difluorophenyl)-3-(5-fluoropyrimidin-4-yl)-1-(1*H*-1,2,4-triazol-1-yl)butan-2-ol, which is represented by Formula (I)

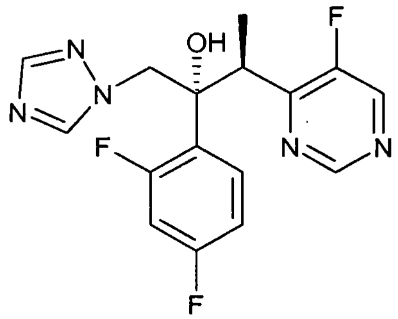

(I)

Voriconazole is disclosed in European Patent EP 0440372B. Precisely, in Example 7 of EP 0440372B, the final product voriconazole is obtained as a crystalline solid, showing a melting point of 127°C and a specific optical rotation of -62° (c = 1 mg/ml). Voriconazole is a commercially marketed pharmaceutically active substance known to be useful for the treatment of some fungal infections.

A problem associated with the use of voriconazole, however, is that voriconazole is practically insoluble in water. Specifically, in Drug Metab. Dispos. 2003, 31, 731, an aqueous solubility of 0.7 mg/ml is reported for voriconazole, which corresponds to a very slightly soluble drug based on the US Pharmacopoeia. Additionally, voriconazole is not stable in water.

In view of the above discussed properties of voriconazole, development of aqueous compositions, in particular aqueous intravenous solutions, with a sufficient shelf life has proved to be difficult. Additionally, development of voriconazole compositions generally with a desirable impurity profile, advantageously prepared by efficient formulation processes has proved problematic.

US 6632803 acknowledges the above discussed low aqueous solubility associated with voriconazole, its instability in water and as such the associated formulation difficulties. US 6632803 further describes that the solubility of voriconazole in water can be increased by molecular encapsulation with a sulphoalkylether cyclodextrin derivative of the type disclosed in International Patent Application WO 91/11172, particularly when n is 5 (a β-cyclodextrin derivative) and the cyclodextrin ring is substituted by sulphobutyl groups. US 6632803 further describes that the aqueous stability of the voriconazole-cyclodextrin derivative complex can be enhanced by lyophilization (freeze-drying). The cyclodextrin derivatives used in formulations according to US 6632803 enable a finished lyophilized product to accommodate high levels of moisture (up to 3.0%) without a detrimental effect on stability. Furthermore, according to US 6632803 the use of such cyclodextrin derivatives controls and minimises the formation of the inactive enantiomer of voriconazole. The specific Example of US 6632803 is as follows, where the sulphobutylether β-cyclodextrin has an average sulphobutylether substitution of 6.5 per cyclodextrin molecule, and each sulphobutylether unit is present as its sodium salt.

Table 1

| *Ingredient* | *mg/ml* |
|---|---|
| Voriconazole | 10.000 |
| Sulphobutylether β-cyclodextrin | 160.000 |
| Water for Injection | to 1.000ml |

Voriconazole is marketed as an I.V. solution under the trademark Vfend™ based on the above described excipients of US 6632803. More specifically, the Vfend™ LV. approved product is a white lyophilized powder containing

nominally 200mg voriconazole and 3200mg sulfobutyl ether β-cyclodextrin sodium in a 30ml Type I clear glass vial. Vfend™ LV. is intended for administration by intravenous infusion. It is a single-dose, unpreserved product. Vials containing 200mg lyophilized voriconazole are intended for reconstitution with water for injection to produce a solution containing 10mg/ml Vfend™ and 160mg/ml of sulfobutyl ether β-cyclodextrin sodium. The resultant solution is further diluted prior to administration to 5mg/ml, or less, prior to administration as an infusion, at a maximum rate of 3mg/kg per hour over 1-2 hours.

CN 1788725 also discloses a voriconazole lyophilized product where the cyclodextrin is a hydroxypropyl-β-cyclodextrin. CN 1788725 also discloses the use of other possible excipients, such as mannitol, lactose, dextran, glucose, glycine, gelatin, povidone or the like, in a voriconazole lyophilized product. The specific Examples of CN 1788725 are, however, primarily based on the general concept of voriconazole hydroxypropyl-β-cyclodextrin and water for injection, although there is also one additional Example that further includes the presense of mannitol. None of the other above mentioned possible excipients are disclosed in a specific Example of CN 1788725.

WO 2006/065726 is essentially concerned with a process of preparing voriconazole, and additionally discloses preparation of solid state voriconazole with a defined impurity profile. Specifically, WO 2006/065726 sets out that for solid state voriconazole there is less than 0.17 area% as measured by HPLC of each of the following impurities: a) 2,4-difluro-1*H*-1-yl-1,2,4-triazolacetophenone; b) 6-ethyl-5-fluoropyrimidine; c) 4-chloro-6-ethyl-5-fluropyrimidine; d) 6-[(1-(5-fluoropyrimidinyl)-6-ethyl]-4-chloro-5-fluoropyrimidine; e) (2R,3S/2S,3R)-3-(4-chloro-5-fluoropyrimidin-6-yl)-2-(2,4-difluorophenyl)-1-(1*H*-1,2,4-triazol-1-yl)-butan-2-ol; f) (2R,3R/2S,3S)-3-(4-chloro-5-fluoropyrimidin-6-yl)-2-(2,4-difluorophenyl)-1-(1*H*-1,2,4-triazol-1-yl)-butan-2-ol; g) (2R,3R/2S,3S)-2-(2,4-difluorophenyl)-3-(5-fluoropyrimidin-4-yl)-1-(1*H*-1,2,4-triazol-1-yl)-butan-2-ol; and h) (2S,3R)-2-(2,4-difluorophenyl)-3-(5-fluoropyrimidin-4-yl)-1-(1*H*-1,2,4-triazol-1-yl)-butan-2-ol. Of particular note from the above list of impurities is 2,4-difluro-1*H*-1-yl-1,2,4-triazolacetophenone, that can also be identified as 2,4-difluoro-2-(1*H*)-1,2,4-triazol-1-yl acetophenone, having the following structure

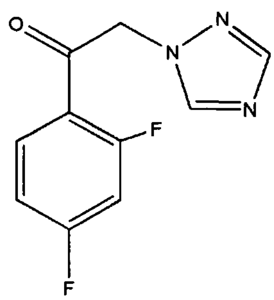

There is, however, no clear or enabling disclosure provided by WO 2006/065726 of an impurity profile associated with a composition of voriconazole, either as prepared during formulation or as present in a pharmaceutical final dosage form.

Patent application US 2002/177511 A1 discloses that glycine-sodium buffers are capable of stabilizing diheterocyclic compounds having a chemical formula different from that of voriconazole.

As referred to above, for example with reference to US 6632803, voriconazole is known to be unstable in water. This instability can be problematic in the preparation and storage of liquid voriconazole preparations that typically employ aqueous carriers, such as water for injection, for example voriconazole concentrates, ready to use voriconazole solutions and lyophilized voriconazole that can be reconstituted in water for injection or a saline solution on administration. There is a need, therefore, to provide aqueous solutions comprising voriconazole with improved properties for pharmaceutical formulation, in particular improved voriconazole stability in an aqueous environment. There is also a need for voriconazole compositions that exhibit a desirable impurity profile, and which can advantageously be prepared by efficient formulation processes. Such compositions, including aqueous solutions, comprising voriconazole, and formulation processes therefor, are now provided by the present invention substantially as hereinafter described in greater detail.

## BRIEF DESCRIPTION OF THE DRAWINGS

Figures 1 and 3 show w/w% amounts of 2,4-difluro-1*H*-1-yl-1,2,4-triazolacetophenone, impurity A, in an aqueous solution according to the present invention comprising voriconazole (1.00g), hydroxypropyl-β-cyclodextrin (12.00g), glycine (2.08g) and water for injection (as required to 100ml), on storage at both room temperature and at 4°C.

Figures 2 and 4 shows an essentially constant voriconazole % amount (based as a % of an initial amount of

voriconazole present) in an aqueous solution according to the present invention comprising voriconazole (1.00g), hydroxypropyl-β-cyclodextrin (12.00g), glycine (2.08g) and water for injection (as required to 100ml), again on storage at both room temperature and at 4°C.

Figure 5 shows w/w% amounts of 2,4-difluro-1*H*-1-yl-1,2,4-triazolacetophenone, impurity A, in an aqueous solution comprising voriconazole (1.00g), hydroxypropyl-β-cyclodextrin (12.00g), sodium chloride (0.9g) and water for injection (as required to 100ml), on storage at both room temperature and at 4°C. The above composition is based on prior art excipients as described in Example 17 of EP 0440372B.

Figure 6 shows a voriconazole % amount (based as a % of an initial amount of voriconazole present) in an aqueous solution comprising voriconazole (1.00g), hydroxypropyl-β-cyclodextrin (12.00g), sodium chloride (0.9g) and water for injection (as required to 100ml), again on storage at both room temperature and at 4°C. Again the above composition is based on prior art excipients as described in Example 17 of EP 0440372B.

Figure 7 depicts the X-ray powder diffractogram (XRD) of voriconazole Form I suitable for use in compositions of the present invention.

Figures 1 and 3 are representative of an impurity profile as associated with an aqueous intermediate solution as provided by the present invention, and specifically an impurity profile associated with an aqueous intermediate solution as obtained during preparation of formulation 1 of Formulation Example 1 hereinafter. Figures 2 and 4 are representative of the observed stability of voriconazole as present in an aqueous intermediate solution as obtained during preparation of formulation 1 of Formulation Example 1 hereinafter. Figures 5 and 6 are corresponding measurements, but for a comparative aqueous solution having excipients as described in Example 17 of EP 0440372B.

Room temperature as referred to above, and also hereinafter, denotes an ambient temperature of surroundings, which is typically understood to be in the range of about 20 to 25°C.

## DETAILED DESCRIPTION OF THE INVENTION

According to the present invention, therefore, there is now provided a composition in the form of a pharmaceutical final dosage form, as defined below, comprising voriconazole, characterized by an impurity profile whereby 2,4-difluro-1*H*-1-yl-1,2,4-triazolacetophenone is present in an amount of less than, or equal to, about 0.17 w/w%, wherein the percentage is referred to the amount of voriconazole in the composition, and wherein the composition further comprises glycine.

There is also provided by the present invention a composition which comprises an aqueous solution comprising voriconazole, which is an intermediate aqueous solution prepared during formulation of a pharmaceutical final dosage form, the composition being characterized by an impurity profile whereby at least 2,4-difluro-1*H*-1-yl-1,2,4-triazolacetophenone is present in an amount of less than, or equal to, about 0.17 w/w%, on storage for 96 hours at room temperature.

There is also provided by the present invention a composition which comprises an aqueous solution comprising voriconazole, characterized in that the product resulting from lyophilization of said composition has a collapse temperature in the range of about -18 to -25°Cwherein the composition further comprises glycine.

In the context of freeze-dried (lyophillised) products the collapse temperature (Tc) is the temperature at which upon heating the freeze-dried product weakens to the point of not being able to support its own structure, leading to incomplete drying, inadequate stability in re-constitution and poor product appearance. Typically the collapse temperature is determined visually by using a cryogenic microscope which is equipped with a thermal cell on the plate of the microscope that allows the reproduction of a lyophilisation process. The structural evolution of the sample on the plate is observed through the lens. This allows the visual detection of sample collapse and the recording of the temperature (Tc) at which collapse takes place.

Preferably, a composition according to the present invention further comprises a cyclodextrin. A composition according to the present invention comprises glycine.

In a preferred embodiment, therefore, the present invention further provides a composition comprising voriconazole, a cyclodextrin and glycine. Preferably, glycine is present in a stabilizing amount.

Where a composition according to the abovementioned preferred embodiment of the present invention comprises a pharmaceutical final dosage form, it is preferred that at least 2,4-difluro-1*H*-1-yl-1,2,4-triazolacetophenone is present in an amount of less than, or equal to, about 0.17ww%. The pharmaceutical final dosage form can be an aqueous solution comprising voriconazole selected from the group consisting of a reconstituted aqueous solution based on a powder composition comprising voriconazole, preferably lyophilized voriconazole; an aqueous concentrate comprising voriconazole; and a ready to use aqueous solution comprising voriconazole. Preferably, the above pharmaceutical final dosage form comprises a reconstituted aqueous solution based on lyophilized voriconazole. Alternatively, the pharmaceutical final dosage form can comprise a powder composition, preferably a lyophilized powder, comprising voriconazole. In the case of a lyophilized powder comprising voriconazole, it is further preferred that this lyophilized powder is further characterized by an impurity profile whereby at least 2,4-difluro-1*H*-1-yl-1,2,4-triazolacetophenone is present in an amount of less than, or equal to, about 0.17w/w%, on storage for three years at room temperature.

Where a composition according to the abovementioned preferred embodiment of the present invention comprises an aqueous solution comprising voriconazole, that is an intermediate aqueous solution prepared during formulation of a pharmaceutical final dosage form, it is preferred that this is characterized by an impurity profile whereby at least 2,4-difluro-1*H*-1-yl-1,2,4-triazolacetophenone is present in an amount of less than, or equal to, about 0.17w/w%, on storage for 96 hours at room temperature. More preferably, the above mentioned impurity profile can be defined as an impurity profile whereby at least 2,4-difluro-1*H*-1-yl- 1,2,4-triazolacetophenone is present in an amount of less than, or equal to, about 0.15w/w%, on storage for 96 hours at room temperature; more preferably less than, or equal to, about 0.14w/w%; more preferably less than, or equal to, about 0.13w/w%; more preferably less than, or equal to, about 0.12w/w%; more preferably less than, or equal to, about 0.11 w/w%; more preferably less than, or equal to, about 0.10w/w%; more preferably less than, or equal to, about 0.09w/w%; more preferably less than, or equal to, about 0.08w/w%; more preferably less than, or equal to, about 0.07w/w%; more preferably less than, or equal to, about 0.06w/w%, on storage for 96 hours at room temperature.

A composition as provided by the present invention can offer advantage at various stages of the formulation process. For example, the present invention can provide an intermediate aqueous solution comprising voriconazole substantially as hereinbefore described that can be stabilized preferably by the presence of glycine again substantially as hereinbefore described and in this way can be stored and / or "held" at various stages of the formulation process for extended periods of time compared to prior art formulation processes, while continuing to exhibit a desirable impurity profile substantially as hereinbefore described. An intermediate solution as provided by the present invention can suitably be further processed into a final dosage form, which can suitably represent a lyophilized product (which is suitable for reconstitution into an aqueous solution), or a reconstituted solution, or an aqueous concentrate, or a ready to use aqueous solution.

Alternatively, the present invention can provide a composition comprising a pharmaceutical final dosage form comprising voriconazole, which similarly can exhibit a desirable impurity profile again substantially as hereinbefore described as a result of a stabilizing effect preferably as provided by glycine. As referred to herein, "final dosage form" can denote a pharmaceutical composition that can be commercially provided and where appropriate may require addition of one or more pharmaceutical excipients prior to administration, such as an aqueous concentrate that may require dilution with an aqueous carrier such as water for injection, or a saline solution, or a lyophilized composition that can require reconstitution with an aqueous carrier. Additionally, "final dosage form" as referred to herein can denote a "ready to use" composition, such as an infusion solution that is in a form suitable for direct administration to a patient, or a reconstituted solution which is based on lyophilized voriconazole and which has been reconstituted with an aqueous carrier.

The desirable impurity profile and / or stability that can in particular be associated with an intermediate aqueous solution comprising voriconazole, prepared during formulation of a voriconazole final dosage form, according to the present invention, can be further illustrated by accompanying Figures 1 to 4, and by comparison thereof with corresponding measurements carried out for compositions based on prior art excipients as illustrated by Figures 5 and 6. In Figures 1 and 3, these show low levels of 2,4-difluro-1*H*-1-yl-1,2,4-triazolacetophenone as an impurity (identified in Figures 1 and 3 as impurity A) on storage at both room temperature and at 4°C for an intermediate aqueous solution comprising voriconazole, prepared during formulation of a voriconazole final dosage form, according to the present invention. In Figures 2 and 4, these show an essentially constant amount of voriconazole again on storage at both room temperature and at 4°C for an intermediate aqueous solution comprising voriconazole, prepared during formulation of a voriconazole final dosage form, according to the present invention. In comparison, however, it can be seen that in Figure 5, this shows increasing amounts of 2,4-difluro-1*H*-1-yl- 1,2,4-triazolacetophenone as an impurity (identified in Figure 5 as impurity A) on storage at room temperature for a solution having prior art excipients as disclosed in Example 17 of EP 0440372B. In particular, 2,4-difluro-1*H*-1-yl-1,2,4-triazolacetophenone (impurity A) increases significantly on storage at room temperature after 72 hours. Also in comparison in Figure 6, this shows decreasing amounts of voriconazole on storage at room temperature for a prior art solution having excipients as disclosed in Example 17 of EP 0440372B. In particular, the amount of voriconazole decreases significantly on storage at room temperature after 72 hours, indicating instability as associated with voriconazole in a solution having prior art excipients as disclosed in Example 17 of EP 0440372B.

The results represented by Figure 1 can be summarized in following Table 2.

Table 2

| Hours | Impurity A w/w% Room temperature | Impurity A w/w% 4°C |
|---|---|---|
| 0 | 0.02 | 0.02 |
| 24 | 0.02 | 0.02 |
| 48 | 0.04 | 0.02 |
| 72 | 0.05 | 0.02 |
| 96 | 0.06 | 0.02 |

The results represented by Figure 2 can be summarized in following Table 3.

Table 3

| Hours | % Voriconazole Room temperature | % Voriconazole 4°C |
|---|---|---|
| 0 | 99.7 | 99.7 |
| 24 | 99.1 | 96.7 |
| 48 | 99.6 | 99.7 |
| 72 | 102.7 | 100.6 |
| 96 | 100.5 | 100.3 |

The results represented by Figure 3 can be summarized in following Table 4.

Table 4

| Hours | Impurity A w/w% Room temperature | Impurity A w/w% 4°C |
|---|---|---|
| 0 | 0.03 | 0.03 |
| 24 | 0.04 | 0.03 |
| 48 | 0.06 | 0.03 |
| 72 | 0.07 | 0.03 |
| 96 | 0.08 | 0.03 |

The results represented by Figure 4 can be summarized in following Table 5.

Table 5

| Hours | % Voriconazole Room temperature | % Voriconazole 4°C |
|---|---|---|
| 0 | 102.8 | 102.8 |
| 24 | 103.0 | 101.1 |
| 48 | 103.9 | 103.6 |
| 72 | 103.6 | 103.7 |
| 96 | 102.9 | 104.1 |

The results represented by Figure 5 can be summarized in following Table 6.

Table 6

| Hours | Impurity A w/w% Room temperature | Impurity A w/w% 4°C |
|---|---|---|
| 0 | 0.04 | 0.04 |
| 24 | 0.07 | 0.04 |

(continued)

| Hours | Impurity A w/w% Room temperature | Impurity A w/w% 4°C |
|---|---|---|
| 48 | 0.13 | 0.05 |
| 72 | 0.11 | 0.05 |
| 96 | 0.28 | 0.04 |

The results represented by Figure 6 can be summarized in following Table 7.

Table 7

| Hours | % Voriconazole Room temperature | % Voriconazole 4°C |
|---|---|---|
| 0 | 104.7 | - |
| 24 | 102.1 | 101.7 |
| 48 | 103.3 | 103.0 |
| 72 | 101.8 | 101.7 |
| 96 | 90.3 | 102.7 |

Typically, in the case where a composition according to the present invention can comprise an aqueous solution then an aqueous carrier as used therein can comprise sterile water for injection. Other aqueous carriers known in the art and suitable for use with IV solutions include aqueous solutions of electrolytes (such as a saline solution) and / or dextrose. In the present invention, an intermediate solution can typically employ water for injection as an aqueous carrier. In the case where the present invention provides a reconstituted solution based on lyophilized voriconazole as a pharmaceutical final dosage form, it is preferred that the aqueous carrier can comprise a saline solution.

An aqueous solution provided as a final dosage form in accordance with the present invention is typically a reconstituted aqueous solution based on lyophilized voriconazole, or an aqueous concentrate comprising voriconazole, or a ready to use aqueous solution comprising voriconazole, which are suitable for LV. administration and where appropriate with a dosing regimen as hereinbefore described for approved VFEND™ LV.. Particularly preferred according to the present invention is a final dosage form which is a reconstituted aqueous solution based on lyophilized voriconazole.

A further particularly preferred pharmaceutical final dosage form according to the present invention comprises a lyophilized composition comprising voriconazole, which can be obtained, or is obtainable, from an intermediate aqueous solution according to the present invention substantially as hereinbefore described. Preferably, a lyophilized composition according to the present invention can be further characterized by an impurity profile substantially as hereinbefore described.

A composition according to the present invention typically includes voriconazole present in an amount of about 0.05 to 2.00w/v %, a cyclodextrin in an amount of about 6.00 to 40.00w/v % and glycine in an amount of about 0.10 to 50.00w/v %. Particularly in such %s, glycine exhibits a stabilizing effect substantially as hereinbefore described.

Typically, voriconazole is present in an amount of about 1.00w/v%. Preferably cyclodextrin is present in an amount of about 9.00 to 40.00w/v%, more preferably in an amount of about 9.00 to 30.00w/v%, more preferably in an amount of about 12.00 to 30.00w/v%. Preferably glycine is present in an amount of about 0.50 to 25.00w/v%, more preferably in an amount of about 1.00 to 10.00w/v%.

Voriconazole suitable for use in a composition according to the present invention can comprise voriconazole prepared according to prior art methods and suitable for pharmaceutical use. Advantageously voriconazole used for the preparation of the compositions of the invention comprises less than, or equal to, about 0.17 w/w%, preferably less than, or equal to, about 0.15 w/w% of 2,4-difluro-1*H*-1-yl-1,2,4-triazolacetophenone wherein the percentage is referred to the amount of voriconazole compound. Preferably, the voriconazole has been subjected to particle size reduction, such as by micronization or the like. Voriconazole of reduced particle size that is suitable for use in a composition according to the present invention can suitably be characterized by a sphericity factor of at least 0.20, more preferably characterized by a sphericity factor in the range of about 0.20 to 0.70, preferably 0.20 to 0.60, preferably 0.20 to 0.50 and even more preferably characterized by a sphericity factor in the range of about 0.22 to 0.60, or 0.22 to 0.50.

Voriconazole suitable for use in a composition according to the present invention can suitably also be characterized by reference to its specific surface area, such as by a specific surface area in the range of about 0.5$m^2$/g to 2$m^2$/g and more preferably characterized by a specific surface area in the range of about 0.9$m^2$/g to 1.7 $m^2$/g and in some embodiments a specific surface area in the range of about 0.9$m^2$/g to 1.6$m^2$/g.

Typically voriconazole suitable for use in a composition according to the present invention is characterized by (i) a sphericity factor of at least 0.20 (more preferably a sphericity factor in the range of about 0.20 to 0.70 and even more preferably a sphericity factor in the range of about 0.22 to 0.60) and (ii) a specific surface area in the range of about 0.5$m^2$/g to 2$m^2$/g (more preferably a specific surface area in the range of about 0.9$m^2$/g to 1.7 $m^2$/g).

Voriconazole suitable for use in a composition according to the present invention can typically be still further characterized by reference to its Sauter diameter. According to the present invention, voriconazole suitable for pharmaceutical use is further characterized by a Sauter diameter in the range of about 4μm to 20μm, and more preferably characterized by a Sauter diameter in the range of about 5μm to 18μm.

Typically, voriconazole suitable for use in a composition according to the present invention can be characterized by (i) a sphericity factor of at least 0.20 (more preferably a sphericity factor in the range of about 0.20 to 0.70 and even more preferably a sphericity factor in the range of about 0.22 to 0.60), and (ii) a specific surface area in the range of about 0.5$m^2$/g to 2$m^2$/g (more preferably a specific surface area in the range of about 0.9$m^2$/g to 1.7 $m^2$/g), and (iii) a Sauter diameter in the range of about 4μm to 20μm (more preferably in the range of about 5μm to 18μm). Alternatively, the voriconazole can be characterized by (i) a specific surface area in the range of about 0.5$m^2$/g to 2$m^2$/g (more preferably a specific surface area in the range of about 0.9$m^2$/g to 1.7 $m^2$/g), and (ii) a Sauter diameter in the range of about 4μm to 20μm (more preferably in the range of about 5μm to 18μm).

Suitably voriconazole suitable for use in a composition according to the present invention is subjected to mechanical size reduction, and even more preferably can be obtained by micronization, milling or any other method known in the art for mechanically reducing the size of particles (i.e. mechanical comminution), such as cutting, chipping, grinding, crushing, trituration and the like, of voriconazole of substantially undefined crystal shape and / or crystal habit. In a preferred embodiment, the present invention employs micronized voriconazole suitable for pharmaceutical use, obtained from voriconazole of substantially undefined crystal shape and / or crystal habit. The term "micronized" as used herein is not intended to limit voriconazole for use in the invention to a particular process for the preparation of voriconazole small particles. The term "micronized" as used herein is thus intended to denote a material formed of small particles, typically voriconazole particles having $D_{90}$ particle size of less than about 250μm, typically less than about 150μm and more typically less than about 100μm. More generally, therefore, and as described above voriconazole small particles suitable for use in a composition according to the present invention can be obtained from any methods known in the art for mechanically reducing the size (i.e. mechanical comminution) of the voriconazole of substantially undefined crystal shape and / or crystal habit, which include any one or more of cutting, chipping, grinding, crushing, milling, micronizing, and trituration. Preferably, the voriconazole small particles are obtained from micronization or milling of voriconazole of substantially undefined crystal shape and / or crystal habit.

As used herein, the wording "substantially undefined crystal shape and / or crystal habit" denotes crystals which are not needle or plate like crystals as described and prepared in IPCOM000125373D. Such "substantially undefined crystal shape and / or crystal habit" can be further understood by reference to its process of preparation and in particular the specific crystallization solvent employed which facilitates the formation of such crystals of "substantially undefined crystal shape and / or crystal habit". Specifically, therefore, voriconazole of "substantially undefined crystal shape and / or crystal habit" as herein described can be further characterized as voriconazole crystallized from a solvent system selected from the group consisting of isopropanol, methyl ethyl ketone / cyclohexane mixture, ethanol / cyclohexane mixture and dimethyl carbonate / cyclohexane mixture, especially isopropanol, and even more suitably as voriconazole crystallized from isopropanol as a crystallization solvent employing a process substantially as described in Voriconazole Preparation Example 1 herein or as described in IPCOM000125373D. By exhibiting a "substantially undefined crystal shape and / or crystal habit" it can be further understood that voriconazole employed as a starting material to yield a voriconazole suitable for use in the present invention, can be further characterized as a material comprising a crystal lattice but with an imperfect or undefined outward shape with reference to regular crystal habits already characterized and defined in the art, in particular an imperfect or undefined outward shape with reference to needle or plate like crystals.

The density (p) of a crystal is a constant value for a specific crystalline form of voriconazole. In this regard, voriconazole suitable for use in a composition according to the present invention corresponds to voriconazole Form I, thus showing an X-ray powder diffractogram substantially identical to the X-ray diffractogram described in IPCOM000125373D and hence substantially identical to the simulated X-ray powder diffractogram calculated from the crystal data reported by K. Ravikumar *et al.* in Acta Cryst. (2007), E63, o565-o567. Thus, the voriconazole suitable for use in a composition according to the present invention can suitably be further characterized by a crystal density value of 1.442g/$cm^3$ at 21°C, a reported value which is constant for all samples of voriconazole Form I. This reported crystal density substantially as hereinbefore described is based on the crystal structure of voriconazole Form I and as such is understood to be an exact further characterizing value for the voriconazole suitable for use in a composition according to the present invention, which typically corresponds to crystalline Form I. It is also understood that the density of a solid can be experimentally measured using a pycnometer, if the crystal structure is not available. However, the true and accurate density refers to mass of solid material divided by its exact volume without porosity, and it can be directly calculated based on the crystal structure of the compound, as determined by X-ray crystallography (see F.M. Richards,

P.F. Lindley, Determination of the density of solids, International Tables for Crystallography, Springer, 2006). Therefore, it is known that the density (p) of the crystal is a constant value for a specific crystalline form of voriconazole.

Voriconazole suitable for use in a composition according to the present invention can suitably be further characterized by an experimental particle density in the range of about 1.300g/cm$^3$ to 1.450g/cm$^3$, more typically a measured experimental particle density in the range of about 1.320g/cm$^3$ to 1.400g/cm$^3$ and even more typically a measured experimental particle density of about 1.36 g/cm$^3$, which values are measured by pycnometry. As is recognized in the art, values for such measured experimental particle density are likely to have some variation from the above discussed exact characterizing value for density based on crystal structure.

Voriconazole suitable for use in a composition according to the present invention can be prepared by a process comprising: (a) providing voriconazole with substantially undefined crystal shape and / or crystal habit substantially as hereinbefore described; and (b) subjecting the voriconazole of step (a) to mechanical particle size reduction (i.e. mechanical comminution) so as to at least modify the sphericity factor and / or specific surface area, and optionally where appropriate also the Sauter diameter and / or $D_{90}$ thereof, thereby providing voriconazole substantially as hereinbefore described.

Typically, mechanical particle size reduction / comminution as referred to in step (b) comprises cutting, chipping, grinding, crushing, milling, micronizing, or triturating the voriconazole of step (a), preferably comprises milling or micronizing, and typically by carrying out milling comprises air-jet-milling or pin-milling.

Preferably, the milling conditions comprise the following steps: (i) milling the voriconazole with undefined shape and/or crystal habit substantially as hereinbefore described; (ii) sieving the milled crystals through a screen of 250μm or less; (iii) separating the sieved crystals; (iv) if there are non-sieved crystals remaining, repeating steps (i) to (iii); and (v) combining the sieved crystals. The milling preferably comprises pin-milling, and more preferably is carried out in a Stainless Steel pin mill working at 7,000rpm. The screen of 250μm or less is preferably a screen of 150μm or less, and more preferably is a screen of 100μm or less.

Suitably voriconazole with substantially undefined crystal shape and / or crystal habit as referred to in step (a) is obtained by crystallizing voriconazole from a solvent system selected from the group consisting of isopropanol, methyl ethyl ketone / cyclohexane mixture, ethanol / cyclohexane mixture and dimethyl carbonate / cyclohexane mixture. Especially preferred is the use of isopropanol.

Typically, a cyclodextrin as present in a composition according to the present invention substantially as hereinbefore described can comprise any cyclodextrin suitable for pharmaceutical use. Preferably, however, the cyclodextrin comprises hydroxypropyl-β-cyclodextrin. It will be recognized in the pharmaceutical field that the inclusion of a cyclodextrin in a pharmaceutical composition can effect an increase in associated toxicity, and as such a reduced amount of impurities, such as at least 2,4-difluro-1*H*-1-yl-1,2,4-triazolacetophenone as being associated with a composition as provided by the present invention thus provides an overall desirable toxicology profile for a composition according to the present invention.

It is herein described processes of preparing a composition according to the present invention substantially as hereinbefore described, said processes not being encompassed by the present invention.

It is herein described a process of preparing an aqueous solution comprising voriconazole, which is an intermediate aqueous solution prepared during formulation of a pharmaceutical final dosage form, which process comprises providing an intermediate aqueous solution comprising voriconazole characterized by an impurity profile whereby at least 2,4-difluro-1*H*-1-yl-1,2,4-triazolacetophenone is present in an amount of less than, or equal to, about 0.17w/w%, on storage for 96 hours at room temperature, said process not being encompassed by the present invention.

A process described herein not according to the present invention further comprises providing glycine in the above described aqueous solution. It is also preferred that a process described herein not according to the present invention further comprises providing a cyclodextrin in the above described aqueous solution. Preferably, the cyclodextrin comprises hydroxypropyl-β-cyclodextrin also as referred to above.

Typically, in a process described herein not according to the present invention voriconazole is included so as to be present in a composition according to the present invention in an amount of about 0.05 to 2.00w/v%, a cyclodextrin in an amount of about 6.00 to 40.00w/v% and glycine in an amount of about 0.10 to 50.00w/v%. Particularly in such %s, glycine exhibits a stabilizing effect substantially as hereinbefore described. More preferably, however, cyclodextrin is included so as to be present in a composition according to the present invention in an amount of about 9.00 to 40.00w/v%, more preferably in an amount of about 9.00 to 30.00w/v%, more preferably in an amount of about 12.00 to 30.00w/v%. More preferably, glycine is included so as to be present in a composition according to the present invention in an amount of about 0.50 to 25.00w/v%, more preferably in an amount of about 1.00 to 10.00w/v.

Typically, a process described herein not according to the present invention further comprises converting an intermediate aqueous solution as described above into a pharmaceutical final dosage form. The pharmaceutical final dosage form can comprise an aqueous solution, such as a reconstituted aqueous solution based on lyophilized voriconazole, or an aqueous concentrate comprising voriconazole, or a ready to use aqueous solution comprising voriconazole, which are suitable for I.V. administration and where appropriate with a dosing regimen as hereinbefore described for

approved VFEND™ I.V. It is preferred that a final dosage form aqueous solution as prepared by a process described herein not according to the present invention is a reconstituted aqueous solution based on lyophilized voriconazole.

It is also further preferred that a process of preparing a final dosage form aqueous solution is carried out in the presence of an inert atmosphere so as to desirably facilitate the provision of a final dosage form aqueous solution having an impurity profile according to the present invention substantially as hereinbefore described.

In the case where a process described herein not according to the present invention is used to further prepare a lyophilized composition and / or a reconstituted aqueous solution based thereon, it is further preferred that an intermediate aqueous solution substantially as described above is further subjected to filtration, filling into sterile vials, subsequently lyophilized and where appropriate reconstituted with an aqueous carrier.

Typically, a lyophilization process employed to prepare a lyophilized composition according to the present invention is characterized by a collapse temperature in the range of about -18 to -25°C. Furthermore, it is typically preferred that a lyophilization process employed to prepare a lyophilized composition according to the present invention is characterized by a sublimation temperature in the range of about -27 to -30°C . A collapse temperature in such a lyophilization process as associated with an aqueous solution according to the present invention can be understood to be energetically favourable compared to, for example, a collapse temperature in a lyophilization process for an aqueous solution based on excipients known from the prior art. In particular, an aqueous solution comprising voriconazole, a cyclodextrin and sodium chloride, which is representative of excipients known from EP 0440372B as hereinbefore discussed, exhibits a collapse temperature of about -37°C in a lyophilization process and as such is associated with further energy input to achieve lyophilization.

There is further provided by the present invention a composition comprising an aqueous solution comprising voriconazole, which has been, or is intended for, lyophilization, which aqueous solution is characterized by a collapse temperature in the range of about -18 to -25°C.

There is still further provided by the present invention a composition comprising an aqueous solution comprising voriconazole, which has been, or is intended for, lyophilization, which aqueous solution is characterized by a sublimation temperature in the range of about -27 to -30°C.

There is still further provided by the present invention a method of using glycine to stabilize a composition comprising voriconazole, which method comprises providing glycine in a stabilizing amount in a composition comprising voriconazole. There is also provided by the present invention a method of using glycine to stabilize a composition comprising an aqueous solution comprising voriconazole, which method comprises providing glycine in a stabilizing amount in an aqueous solution comprising voriconazole. There is also provided by the present invention a method of using glycine to stabilize a composition comprising a lyophilized final dosage form comprising voriconazole, which method comprises providing glycine in a stabilizing amount in said lyophilized final dosage form. In the case of a final dosage form, it is preferred that glycine stabilizes the same whereby at least 2,4-difluro-1H-1-yl-1,2,4-triazolacetophenone is present in an amount of less than, or equal to, about 0.17 w/w%. In the case of an intermediate aqueous solution prepared during formulation of a voriconazole final dosage form, it is preferred that glycine stabilizes the same whereby at least 2,4-difluro-1*H*-1-yl-1,2,4-triazolacetophenone is present in an amount of less than, or equal to, about 0.17 w/w%, on storage for 96 hours at room temperature.

Voriconazole compositions as provided in accordance with the present invention are suitable for use in the treatment of fungal infections by administering to the patient an effective amount of a voriconazole composition in accordance with the present invention substantially as hereinbefore described.

The term "about" when used in the present invention preceding a number and referring to it, is meant to designate any value which lies within the range defined by the number ±10% of its value, preferably a range defined by the number ±5%, more preferably range defined by the number ±2%, still more preferably a range defined by the number ±1%. For example "about 10" should be construed as meaning within the range of 9 to 11, preferably within the range of 9.5 to 10.5, more preferably within the range of 9.8 to 10.2, and still more preferably within the range of 9.9 to 10.1

The present invention will now be further illustrated by reference to the following Examples, which do not limit the scope of the invention in any way.

## EXAMPLES:

### General experimental conditions for characterization of voriconazole suitable for use in aqueous solutions of the invention

### Particle Size Distribution Method:

The particle size measurement for voriconazole was obtained using a Malvern Mastersizer S particle size analyzer equipped with a 2 milliwatt Helium/Neon laser and a Fourier Transform lens system. The sample was run using the 2.40mm lens. The sample unit was a MS1-Small Volume Sample Dispersion Unit stirred cell. The dispersant was deionized water. The sample particle size distribution was assumed to follow a normal distribution.

Analysis model: polydisperse. Setup presentation: 3_WATERF. Particle R.I. = (1.6800, 0.01). Dispersant R.I. = 1.33.

Samples for analysis were prepared by wetting a weighed amount of voriconazole (approximately 250mg) with 20mL of a 0.1% solution of Tween 20 in deionized water. This sample was sonicated for 2 minutes and delivered dropwise to the previously background and corrected measuring cell filled with dispersant (deionized water) until the obscuration reached the desired level. Volume distributions were obtained for at least six measures. For characterization, the values of $D_{10}$, $D_{50}$ and $D_{90}$ (by volume), D[4,3] (mean diameter by volume) and D[3,2] (mean diameter by surface area to volume, or Sauter diameter) were specifically listed, each one being the mean of the measured values available for each characterization parameter.

The notation $D_X$ [also written as D(v, 0.X)] means that X% of the particles have a diameter less than a specified diameter D. Thus a $D_{90}$ [or D(v, 0.9)] of 100μm means that 90% of the particles have a diameter less than 100μm.

Optical Microscopy:

A solid sample (containing voriconazole crystals) was mounted on a slide and analyzed using an Olympus BX41 microscope. The micrographs were taken at 40X magnification.

Specific Surface Area Method:

The BET (Brunauer, Emmett and Teller) specific surface area for voriconazole was measured using a Micromeritics™ GEMINI V equipment (GEMINI CONFIRM V2.00 Software™). The sample for analysis was degassed at 30°C for 10 minutes and at 80°C for one hour. The determination of the adsorption of $N_2$ at 77 K was measured for relative pressures in the range of 0.02 to 0.2 for a weighed amount of voriconazole (i.e., approximately 0.5g).

Density (Pycnometry):

Density of voriconazole samples was determined at 25°C using a 50mL glass pycnometer. A pre-weighted amount of about 1g of voriconazole was introduced in the pycnometer, and the volume was filled with n-heptane, where voriconazole is practically insoluble at the working temperature. The density of the voriconazole sample *($\rho s$)* can be determined from the known density of n-heptane ($\rho_H$: 0.685g/cm$^3$), the weight of the pycnometer filled only with n-heptane ($W_H^0$), the weight of the filled pycnometer containing both voriconazole and n-heptane ($W_{S+H}$), and the weight of voriconazole ($W_S$):

$$\rho_S = \frac{W_S \cdot \rho_H}{W_H^0 + W_S - W_{S+H}}$$

Density of voriconazole samples was determined for three times, being the listed result the mean of the three values available for each sample.

X-ray Powder Diffraction (XRD):

The X-ray diffractogram was obtained using a RX SIEMENS D5000 diffractometer with a vertical goniometer and a copper anodic tube, radiation $CuK_\alpha$, λ= 1, 54056Å.

HPLC Method:

The chromatographic separation was carried out in a Symmetry C18, 3.5μm, 4.6mm x 150mm column.

The mobile phase A was a 0.010M ammonium formate buffer, pH 4.0, which was prepared from 0.63g of HCOONH4 dissolved in 1000mL of water, adjusting pH to 4.0 with formic acid. The mobile phase was mixed and filtered through a 0.22μm nylon membrane under vacuum.

The mobile phase B was acetonitrile.

The chromatograph was programmed as follows:

Initial 0-13 min. 75% mobile phase A, 13-25 min. linear gradient to 40% mobile phase A, 25-35 min. isocratic 40%

mobile phase A, 35-40 min. linear gradient to 25% mobile phase A, 40-55 min. isocratic 25% mobile phase A, 55-60 min. linear gradient to 75% mobile phase A and 60-65 min. equilibration with 75% mobile phase A.

The chromatograph was equipped with a 254nm detector and the flow rate was 1.0mL/min at 20-25°C.

Example 1:

6.32Kg of voriconazole were dissolved with 16.6L of isopropanol at reflux temperature. The solution was cooled down to 0-2°C and stirred at that temperature for 1 hour. The resulting suspension was filtered, washing the cake with 2.5L of isopropanol. After drying, 4.90Kg of a white solid was obtained (77.5% yield) corresponding to voriconazole. Optical microscopy: Crystals with undefined crystal shape. XRD: Form I, see Figure 7.

Example 2:

Voriconazole in form of crystals with undefined shape as obtained in Example 1 was micronized by using a Stainless Steel TL micronizer working at 5 bar Venturi pressure and 3 bar mill pressure. Density (pycnometry): 1.35g/cm$^3$. Particle Size Distribution: $D_{10}$: 7.4μm, $D_{50}$: 29.7μm, $D_{90}$: 102.8μm; D[4,3]: 44.5μm; D[3,2]: 15.4μm. Specific Surface Area (BET): 0.9319m$^2$/g. Optical microscopy: Micronized product.

Examples 3 to 6:

Voriconazole in form of crystals with undefined crystal shape as obtained in Example 1 was micronized by using a Stainless Steel TL micronizer working at 5 bar Venturi pressure and 3 bar mill pressure.

Results for 4 different batches are summarized in Table 8.

Table 8

| Example | Measured Density (g/cm$^3$) | Particle Size Distribution (μm) | | | | | Specific Surface Area (m$^2$/g) |
|---|---|---|---|---|---|---|---|
| | | $D_{10}$ | $D_{50}$ | $D_{90}$ | D[4,3] | D[3,2] | |
| 3 | 1.33 | 6.0 | 21.7 | 56.2 | 27.3 | 12.2 | 1.0997 |
| 4 | 1.32 | 6.0 | 22.4 | 64.0 | 30.3 | 12.3 | 1.0992 |
| 5 | 1.37 | 7.6 | 32.3 | 92.7 | 42.8 | 15.8 | 1.1451 |
| 6 | 1.40 | 8.6 | 33.4 | 92.3 | 43.3 | 17.2 | 0.9769 |

Example 7

Voriconazole in form of crystals with undefined crystal shape as obtained in Example 1 was milled and sieved through a 100μm screen. The sieved crystals were separated and the rest of crystals were milled and sieved again through a 100μm screen. This milling and sieving was repeated with the rest of crystals (i.e. crystals not passing through the 100μm sieve screen) until all crystals were sieved through the 100μm screen. Finally, the sieved crystals were combined. The milling was carried out by using a Stainless Steel pin mill, working at 7,000rpm. Particle Size Distribution: $D_{10}$: 7.5μm, $D_{50}$: 25.3μm, $D_{90}$: 57.4μm; D[4,3]: 24.9μm; D[3,2]: 14.3μm; Specific Surface Area (BET): 1.1032m$^2$/g.

Example 8

Voriconazole as obtained in Example 7 was micronized twice by using a Stainless Steel TL micronizer working at 7 bar Venturi pressure and 5 bar mill pressure. Particle Size Distribution: $D_{10}$: 2.5μm, $D_{50}$: 7.3μm, $D_{90}$: 29.9μm; D[4,3]: 14.2μm; D[3,2]: 5.2μm; Specific Surface Area (BET): 1.5910m$^2$/g.

Example 9:

Sphericity factors for some batches of micronized voriconazole obtained from crystals with undefined shape are calculated using either the measured particle density value or the reported crystal density value and are summarized in Tables 9 and 10, respectively.

Table 9

| Example | $\rho$ (g/cm$^3$) | $D_{MVS}$ ($\mu$m) | $S_w$ (m$^2$/g) | $^a\Psi_w$ |
|---|---|---|---|---|
| 2 | 1.35 | 15.4 | 0.9319 | 0.31 |
| 3 | 1.33 | 12.2 | 1.0997 | 0.34 |
| 4 | 1.32 | 12.3 | 1.0992 | 0.34 |
| 5 | 1.37 | 15.8 | 1.1451 | 0.24 |
| 6 | 1.40 | 17.2 | 0.9769 | 0.25 |
| [a]Value calculated using the measured particle density value. | | | | |

Table 10

| Example | $\rho$ (g/cm$^3$) | $D_{MVS}$ ($\mu$m) | $S_w$ (m$^2$/g) | $^b\Psi_w$ |
|---|---|---|---|---|
| 2 | 1.442 | 15.4 | 0.9319 | 0.29 |
| 3 | 1.442 | 12.2 | 1.0997 | 0.31 |
| 4 | 1.442 | 12.3 | 1.0992 | 0.31 |
| 5 | 1.442 | 15.8 | 1.1451 | 0.23 |
| 6 | 1.442 | 17.2 | 0.9769 | 0.23 |
| 7 | 1.442 | 14.3 | 1.1032 | 0.26 |
| 8 | 1.442 | 5.2 | 1.5910 | 0.50 |
| [b]Value calculated using the reported crystal density value. | | | | |

As is recognized in the art and substantially as hereinbefore discussed, values for measured particle density are likely to have some variation from the above discussed exact characterizing value for density based on crystal structure, and a person skilled in the art would recognize that sphericity could be calculated based on either value. The inventors prefer, however, to base sphericity on the exact characterizing value for density based on crystal structure.

<u>Formulation Example:</u>

<u>Example 1:</u>

Formulation 1: (12% Hydroxypropyt-β-cyclodextrin):

Table 11

| *Ingredient* | *Amount (g)* |
|---|---|
| Voriconazole | 1.00 |
| Hydroxypropyl-β-cyclodextrin | 12.00 |
| Glycine | 2.08 |
| Water for Injection | to 100ml |

Formulation 2: (30% Hydroxypropyl-β-cyclodextrin):

Table 12

| *Ingredient* | *Amount (g)* |
|---|---|
| Voriconazole | 1.00 |
| Hydroxypropyl-β-cyclodextrin | 30.00 |

(continued)

| *Ingredient* | *Amount (g)* |
|---|---|
| Glycine | 2.09 |
| Water for Injection | to 100ml |

Comparative Formulation 3: (9% Hydroxypropyl-β-cyclodextrin):

Table 13

| *Ingredient* | *Amount (g)* |
|---|---|
| Voriconazole | 1.00 |
| Hydroxypropyl-β-cyclodextrin | 9.00 |
| Water for Injection | to 100ml |

Process:

Hydroxypropyl-β-cyclodextrin was dissolved in 80% of theoretical weight of water for injection. The mixture was stirred for about 1 minute with a magnetic stirrer until dissolution was complete at room temperature. The resulting solution was heated to about 30°C±4°C. Voriconazole was weighed and added to the above solution, while maintaining the temperature at about 30°C±4°C until dissolution of voriconazole was complete. Glycine was weighed and added to the above solution. Heating was stopped. The solution was cooled to about 20-25°C before adding water for injection as required to obtain a final volume of 100ml. The pH and density of the resulting solution were measured. The solution was filtered through PVDF 0.22μm filters and filled in glass type I vials. Lyophilization was carried out.

## Claims

1. A composition in the form of a pharmaceutical final dosage form selected from the group consisting of a reconstituted aqueous solution based on lyophilized voriconazole, or an aqueous concentrate comprising voriconazole, or a ready to use aqueous solution comprising voriconazole, or a lyophilized composition comprising voriconazole, or a powder composition comprising voriconazole, **characterized by** an impurity profile whereby 2,4-difluro-1*H*-1-yl-1,2,4-triazolacetophenone is present in an amount of less than, or equal to, about 0.17 w/w%, wherein the percentage is referred to the amount of voriconazole in the composition, and wherein the composition further comprises glycine.

2. A composition which comprises an aqueous solution comprising voriconazole, **characterized in that** the product resulting from lyophilization of said composition has a collapse temperature in the range of -18 to -25°C, wherein the composition further comprises glycine.

3. A composition according to any of claims 1 to 2, which further comprises a cyclodextrin.

4. A composition comprising voriconazole, a cyclodextrin and glycine.

5. A composition according to claim 4, **characterized by** an impurity profile whereby 2,4-difluro-1*H*-1-yl-1,2,4-triazolacetophenone is present in an amount of less than, or equal to, about 0.17 w/w%, wherein the percentage is referred to the amount of voriconazole in the composition.

6. A composition according to claim 1 or 5, either comprising lyophilized powder or in the form of an aqueous solution comprising voriconazole selected from the group consisting of a reconstituted aqueous solution based on lyophilized voriconazole, an aqueous concentrate comprising voriconazole and a ready to use aqueous solution comprising voriconazole.

7. A composition according to claims 1 or 6, **characterized in that** it comprises a lyophilized powder and further **characterized by** an impurity profile whereby 2,4-difluro-1*H*-1-yl-1,2,4-triazolacetophenone is present in an amount of less than, or equal to, about 0.17w/w%, on storage for three years at room temperature, wherein the percentage is referred to the amount of voriconazole in the composition.

8. A composition according to claim 4, in the form of an intermediate aqueous solution prepared during formulation of a pharmaceutical final dosage form.

9. A composition according to any of claims 1 to 8, which comprises hydroxypropyl-β-cyclodextrin.

10. A composition according to any of claims 1 to 9, which comprises voriconazole present in an amount of 0.05 to 2.00w/v%, preferably in an amount of about 1.00 w/v, a cyclodextrin present in an amount of 6.00 to 40.00w//w%, preferably in an amount of 9.00 to 40.00w/v%, more preferably in an amount of 9.00 to 30.00w/v%, more preferably preferably in an amount of 12.00 to 30.00w/v% and glycine present in an amount of 0.10 to 50.00w/v%, preferably in an amount of 0.50 to 25.00w/v%, more preferably 1.00 to 10.00w/v%.

11. A method of using glycine to stabilize a composition comprising voriconazole, in particular an aqueous composition or a lyophilized composition, which method comprises providing glycine in a stabilizing amount in a composition comprising voriconazole.

**Patentansprüche**

1. Zusammensetzung in Form einer endgültigen pharmazeutischen Darreichungsform ausgewählt aus der Gruppe bestehend aus einer rekonstituierten wässrigen Lösung auf der Basis von lyophilisiertem Voriconazol, oder einem wässrigen Konzentrat umfassend Voriconazol, oder einer gebrauchsfertigen wässrigen Lösung umfassend Voriconazol, oder einer lyophilisierten Zusammensetzung umfassend Voriconazol, oder einer Pulverzusammensetzung umfassend Voriconazol, **gekennzeichnet durch** ein Verunreinigungsprofil, wobei 2,4-Difluro-1*H*-1-yl-1,2,4-triazolacetophenon in einer Menge von weniger als oder gleich ungefähr 0,17 Gew./Gew.-% vorhanden ist, wobei sich der Prozentsatz auf die Menge von Voriconazol in der Zusammensetzung bezieht, und wobei die Zusammensetzung außerdem Glycin umfasst.

2. Zusammensetzung, welche eine wässrige Lösung umfassend Voriconazol umfasst, **dadurch gekennzeichnet, dass** das aus der Lyophilisierung der Zusammensetzung resultierende Produkt eine Kollapstemperatur im Bereich von -18 bis -25°C aufweist, wobei die Zusammensetzung außerdem Glycin umfasst.

3. Zusammensetzung nach einem der Ansprüche 1 bis 2, welche außerdem ein Cyclodextrin umfasst.

4. Zusammensetzung umfassend Voriconazol, ein Cyclodextrin und Glycin.

5. Zusammensetzung nach Anspruch 4, **gekennzeichnet durch** ein Verunreinigungsprofil, wobei 2,4-Difluro-1*H*-1-yl-1,2,4-triazolacetophenon in einer Menge von weniger als oder gleich ungefähr 0,17 Gew./Gew.-% vorhanden ist, wobei sich der Prozentsatz auf die Menge von Voriconazol in der Zusammensetzung bezieht.

6. Zusammensetzung nach Anspruch 1 oder 5, entweder umfassend lyophilisiertes Pulver oder in Form einer wässrigen Lösung umfassend Voriconazol ausgewählt aus der Gruppe bestehend aus einer rekonstituierten wässrigen Lösung auf der Basis von lyophilisiertem Voriconazol, einem wässrigen Konzentrat umfassend Voriconazol und einer gebrauchsfertigen wässrigen Lösung umfassend Voriconazol.

7. Zusammensetzung nach den Ansprüchen 1 oder 6, **dadurch gekennzeichnet, dass** sie ein lyophilisiertes Pulver umfasst und außerdem **gekennzeichnet durch** ein Verunreinigungsprofil, wobei 2,4-Difluro-1*H*-1-yl-1,2,4-triazolacetophenon in einer Menge von weniger als oder gleich ungefähr 0,17 Gew./Gew.-% vorhanden ist, nach Aufbewahrung während drei Jahren bei Raumtemperatur, wobei sich der Prozentsatz auf die Menge von Voriconazol in der Zusammensetzung bezieht.

8. Zusammensetzung nach Anspruch 4, in Form einer intermediären wässrigen Lösung, die während der Formulierung einer endgültigen pharmazeutischen Darreichungsform hergestellt wird.

9. Zusammensetzung nach einem der Ansprüche 1 bis 8, welche Hydroxypropyl-β-cyclodextrin umfasst.

10. Zusammensetzung nach einem der Ansprüche 1 bis 9, welche Voriconazol, das in einer Menge von 0,05 bis 2,00 Gew./Vol.-%, vorzugsweise in einer Menge von ungefähr 1,00 Gew./Vol. vorhanden ist, ein Cyclodextrin, das in einer Menge von 6,00 bis 40,00 Gew./Gew.-%, vorzugsweise in einer Menge von 9,00 bis 40,00 Gew./Vol.-%,

stärker bevorzugt in einer Menge von 9,00 bis 30,00 Gew./Vol.-%, noch stärker bevorzugt in einer Menge von 12,00 bis 30,00 Gew./Vol.-% vorhanden ist, und Glycin, das in einer Menge von 0,10 bis 50,00 Gew./Vol.-%, vorzugsweise in einer Menge von 0,50 bis 25,00 Gew./Vol.-%, stärker bevorzugt 1,00 bis 10,00 Gew./Vol.-% vorhanden ist, umfasst.

**11.** Verfahren zum Verwenden von Glycin zum Stabilisieren einer Zusammensetzung umfassend Voriconazol, insbesondere einer wässrigen Zusammensetzung oder einer lyophilisierten Zusammensetzung, wobei das Verfahren das Bereitstellen von Glycin in einer stabilisierenden Menge in einer Zusammensetzung umfassend Voriconazol umfasst.

**Revendications**

**1.** Composition sous la forme d'une forme posologique finale pharmaceutique sélectionnée dans le groupe constitué par une solution aqueuse reconstituée à base de voriconazole lyophilisé, ou un concentré aqueux comprenant du voriconazole, ou une solution aqueuse prête à l'emploi comprenant du voriconazole, ou une composition lyophilisée comprenant du voriconazole, ou une composition poudreuse comprenant du voriconazole, **caractérisée par** un profil d'impureté selon lequel la 2,4-difluoro-1*H*-1-yl-1,2,4-triazolacétophénone est présente en une quantité inférieure, ou égale, à environ 0,17 % p/p, dans laquelle le pourcentage se rapporte à la quantité de voriconazole dans la composition, et dans laquelle la composition comprend en outre de la glycine.

**2.** Composition qui comprend une solution aqueuse comprenant du voriconazole, **caractérisée en ce que** le produit résultant de la lyophilisation de ladite composition a une température de collapse dans la plage de -18 à -25°C, dans laquelle la composition comprend en outre de la glycine.

**3.** Composition selon l'une quelconque des revendications 1 à 2, qui comprend en outre une cyclodextrine.

**4.** Composition comprenant du voriconazole, une cyclodextrine et de la glycine.

**5.** Composition selon la revendication 4, **caractérisée par** un profil d'impureté selon lequel la 2,4-difluoro-1*H*-1-yl-1,2,4-triazolacétophénone est présente en une quantité inférieure, ou égale, à environ 0,17 % p/p, dans laquelle le pourcentage se rapporte à la quantité de voriconazole dans la composition.

**6.** Composition selon la revendication 1 ou 5, soit comprenant une poudre lyophilisée soit sous la forme d'une solution aqueuse comprenant du voriconazole sélectionnée dans le groupe constitué par une solution aqueuse reconstituée à base de voriconazole lyophilisé, un concentré aqueux comprenant du voriconazole et une solution aqueuse prête à l'emploi comprenant du voriconazole.

**7.** Composition selon les revendications 1 ou 6, **caractérisée en ce qu'**elle comprend une poudre lyophilisée et **caractérisée en outre par** un profil d'impureté selon lequel la 2,4-difluoro-1*H*-1-yl-1,2,4-triazolacétophénone est présente en une quantité inférieure, ou égale, à environ 0,17 % p/p, en conservation pendant trois ans à température ambiante, dans laquelle le pourcentage se rapporte à la quantité de voriconazole dans la composition.

**8.** Composition selon la revendication 4, sous la forme d'une solution aqueuse intermédiaire préparée pendant la formulation d'une forme posologique finale pharmaceutique.

**9.** Composition selon l'une quelconque des revendications 1 à 8, qui comprend de l'hydroxypropyl-$\beta$-cyclodextrine.

**10.** Composition selon l'une quelconque des revendications 1 à 9, qui comprend du voriconazole présent en une quantité de 0,05 à 2,00 % p/v, de préférence en une quantité d'environ 1,00 p/v, une cyclodextrine présente en une quantité de 6,00 à 40,00 % p/v, de préférence en une quantité de 9,00 à 40,00 % p/v, plus préférablement en une quantité d'environ 9,00 à 30,00 % p/v, plus préférablement en une quantité de 12,00 à 30,00 % p/v et de la glycine présente en une quantité de 0,10 à 50,00 % p/v, de préférence en une quantité de 0,50 à 25,00 % p/v, plus préférablement de 1,00 à 10,00 % p/v.

**11.** Procédé d'utilisation de la glycine pour stabiliser une composition comprenant du voriconazole, en particulier une composition aqueuse ou une composition lyophilisée, lequel procédé comprend l'apport de glycine en une quantité de stabilisation dans une composition comprenant du voriconazole.

Figure 1:

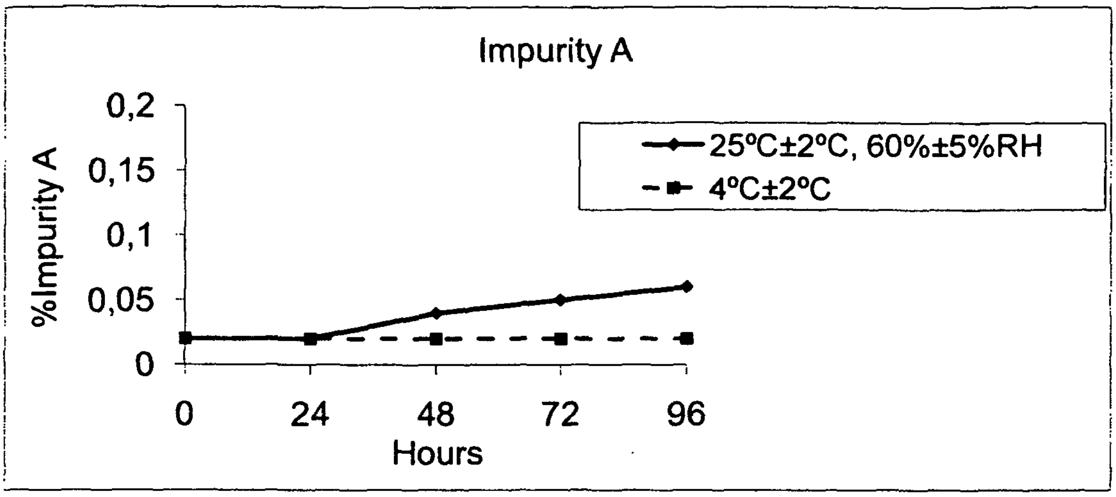


Figure 2:

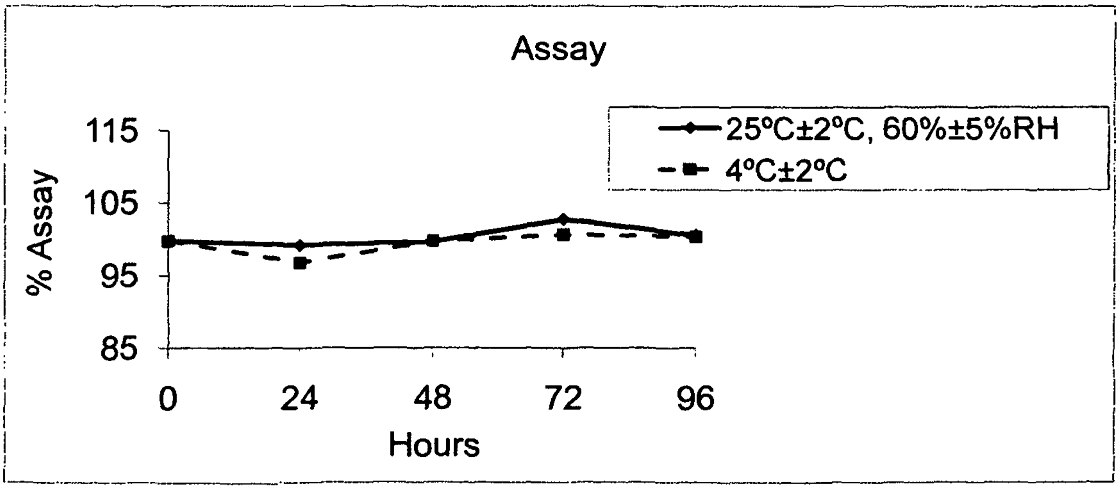

Figure 3:

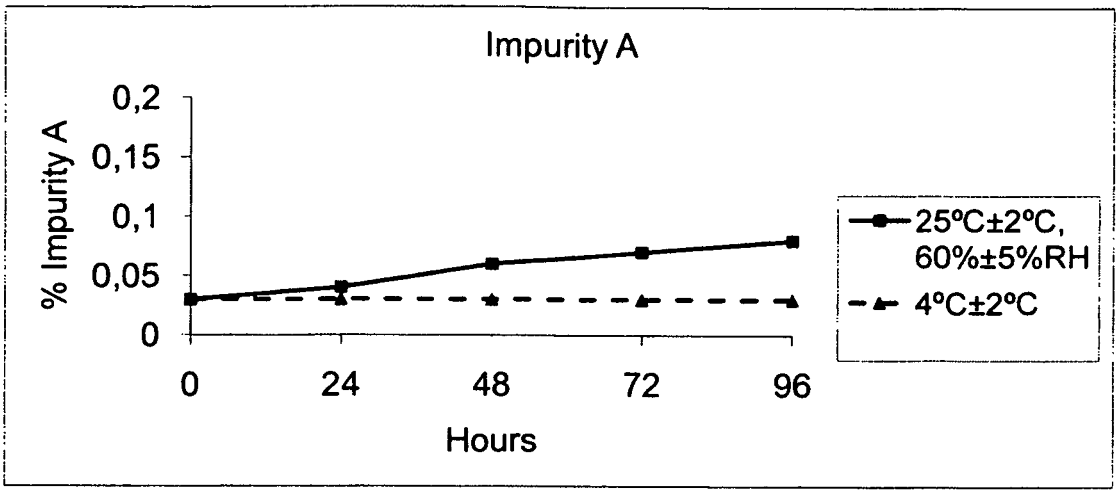


Figure 4:

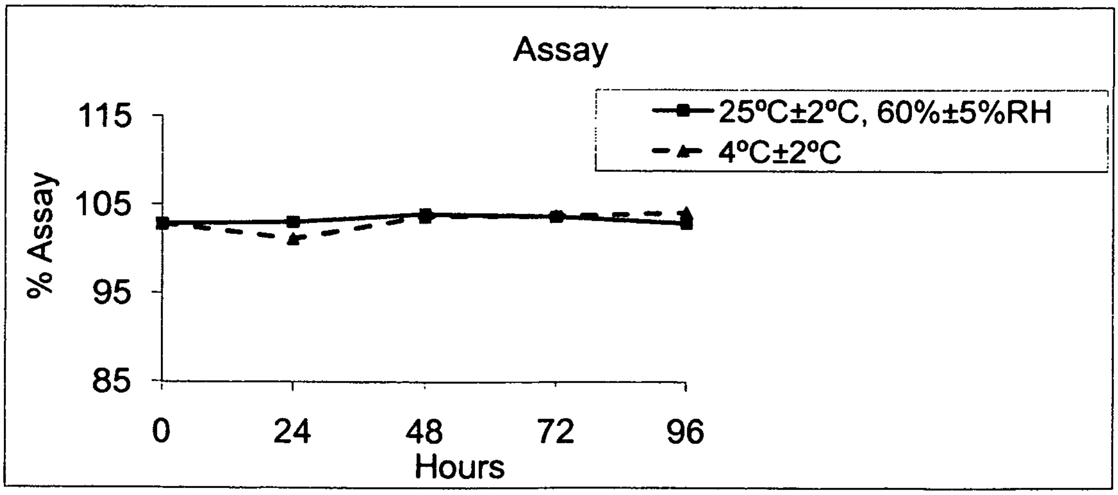

Figure 5:

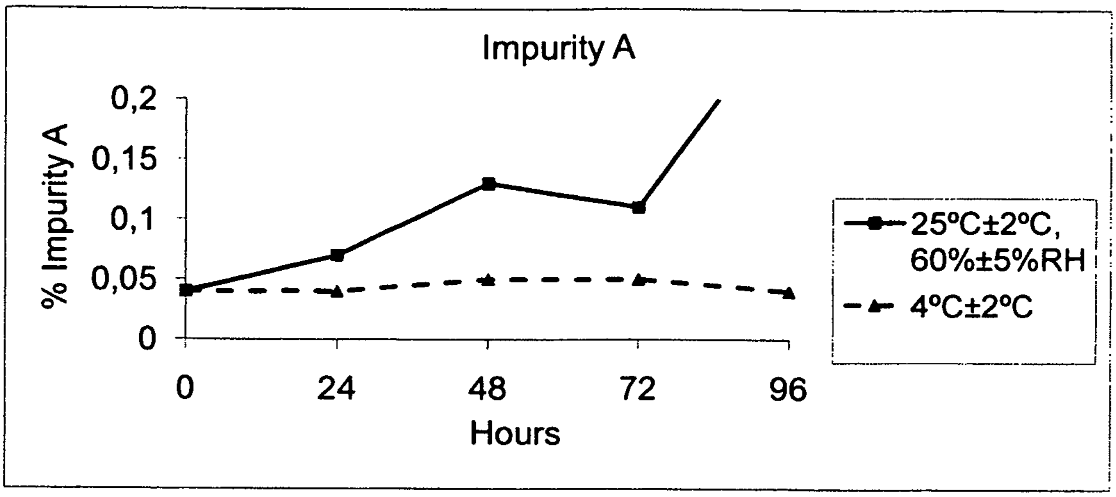


Figure 6:

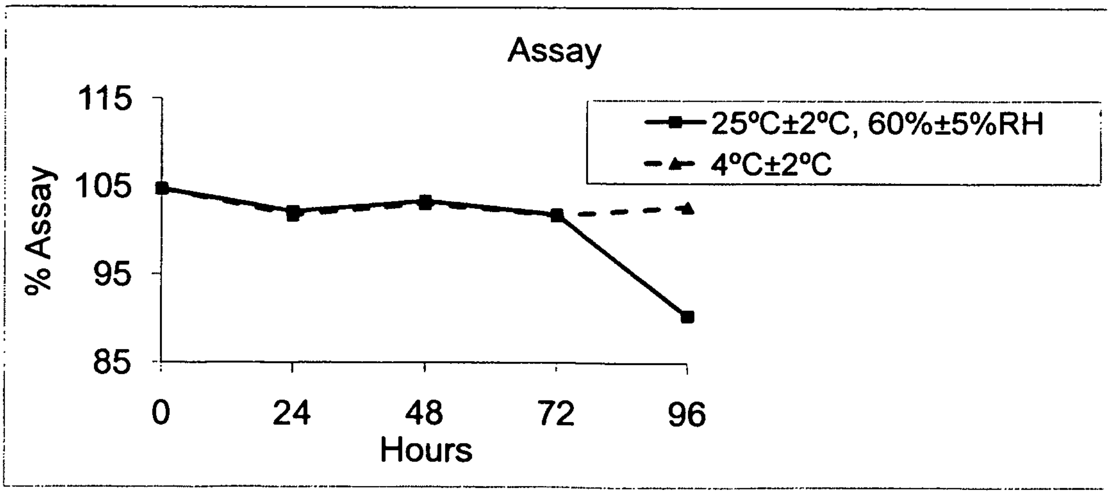

Figure 7

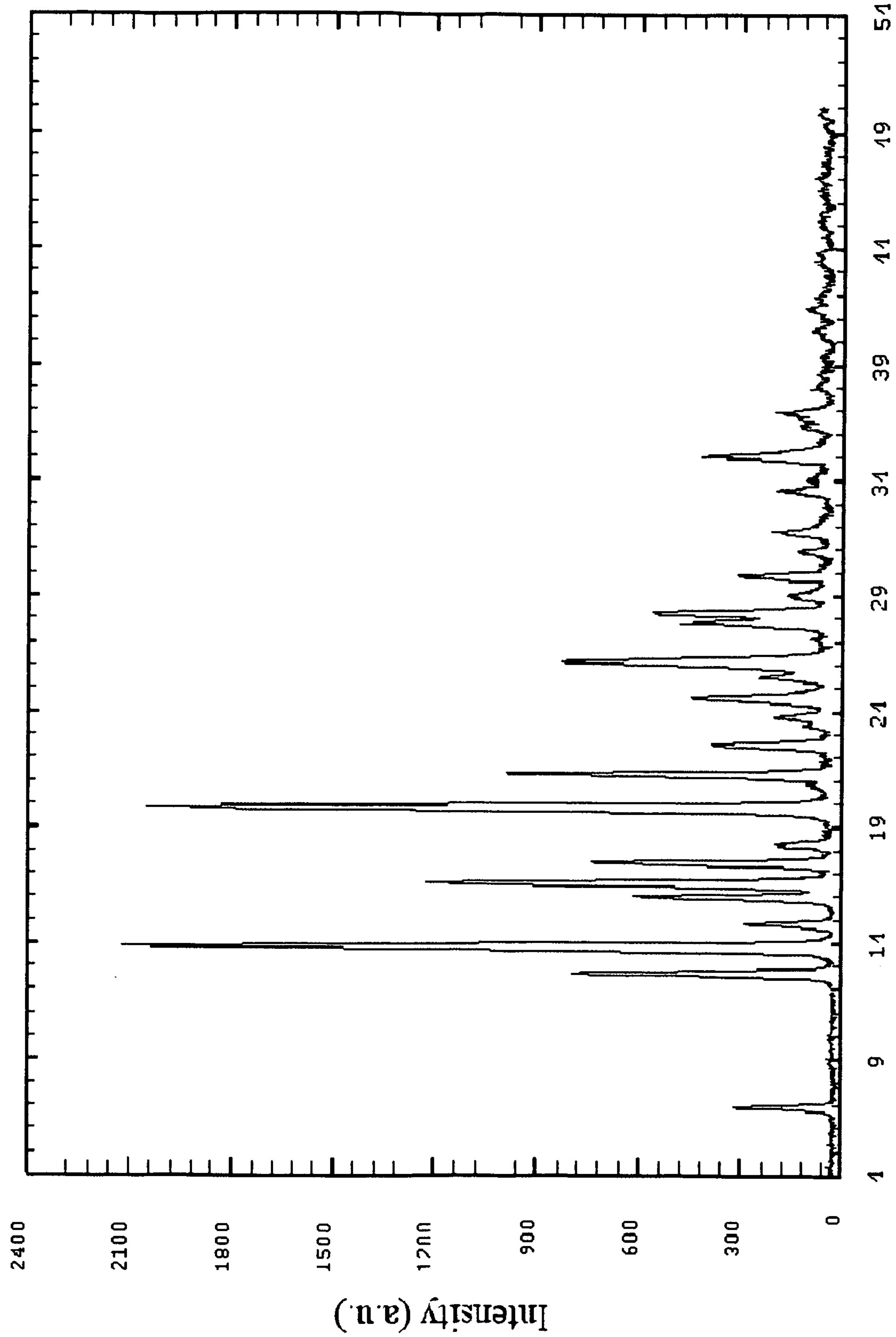

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0440372 B
- US 6632803 B
- WO 9111172 A
- CN 1788725
- WO 2006065726 A
- US 2002177511 A1

### Non-patent literature cited in the description


- *Drug Metab. Dispos,* 2003, vol. 31, 731
- **F.M. RICHARDS ; P.F. LINDLEY.** Determination of the density of solids, International Tables for Crystallography. Springer, 2006